(19) Europäisches Patentamt
European Patent Office
Office européen des brevets

(11) Veröffentlichungsnummer: **0 271 833 B1**

(12) # EUROPÄISCHE PATENTSCHRIFT

(45) Veröffentlichungstag der Patentschrift: **07.09.94**

(51) Int. Cl.[5]: **C07D 239/52**, C07D 239/56, C07D 239/58

(21) Anmeldenummer: **87118369.5**

(22) Anmeldetag: **11.12.87**

Die Akte enthält technische Angaben, die nach dem Eingang der Anmeldung eingereicht wurden und die nicht in dieser Patentschrift enthalten sind.

(54) **Verfahren zur Herstellung von Aminopyrimidinen.**

(30) Priorität: **16.12.86 DE 3642832**

(43) Veröffentlichungstag der Anmeldung:
**22.06.88 Patentblatt 88/25**

(45) Bekanntmachung des Hinweises auf die Patenterteilung:
**07.09.94 Patentblatt 94/36**

(84) Benannte Vertragsstaaten:
**CH DE FR GB IT LI NL**

(56) Entgegenhaltungen:
**EP-A- 0 024 200**

(73) Patentinhaber: **HOECHST SCHERING AGREVO GmbH**
**Miraustrasse 54**
**D-13509 Berlin (DE)**

(72) Erfinder: **Lachhein, Stephen, Dr.**
**Kiedricher Strasse 18**
**D-6238 Hofheim am Taunus (DE)**

EP 0 271 833 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Herstellung von Pyrimidinen der Formel I

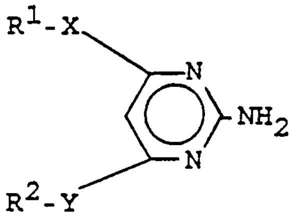

I,

worin

X und Y unabhängig voneinander Sauerstoff oder Schwefel und

$R^1$ und $R^2$ unabhängig voneinander $(C_1\text{-}C_4)$Alkyl, $(C_1\text{-}C_4)$Alkoxy-$(C_1\text{-}C_2)$-alkyl oder Halo$(C_1\text{-}C_4)$alkyl bedeuten,

durch Umsetzung eines Propandiimidates der Formel II oder eines seiner Salze,

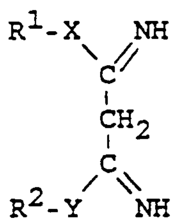

II

worin $R^1$ und $R^2$ wie in Formel I definiert sind, mit einem Cyansäurederivat, dadurch gekennzeichnet, daß man mit einem Cyansäureester der Formel III als Cyansäurederivat,

$R^3 - O - C \equiv N$ III

worin

$R^3$ Phenyl, Phenyl, das durch 1, 2 oder 3 Reste aus den Resten Halogen, $(C_1\text{-}C_4)$Alkoxy und $(C_1\text{-}C_4)$Alkyl substituiert ist, oder $\alpha$- oder $\beta$-Naphthyl bedeutet,

in einem inerten organischen Lösungsmittel bei Reaktionstemperaturen von 0-180 °C umsetzt.

Verbindungen der Formel I sind wertvolle Zwischenprodukte bei der Herstellung von Sulfonylharnstoffen mit berbizider Wirkung; siehe beispielsweise US-A-4,169,719 oder EP-A-071 958 (US-A-4,492,598).

Bekannt ist die Umsetzung von Nucleophilen und Dinucleophilen mit Cyansäureestern (E. Grigat, Angew. Chem. 84, 1008 (1972); Angew. Chem. Int. Ed. Vol 11, 949 (1972)).

Ferner ist bekannt, daß Pyrimidine der Formel I durch Umsetzung von Propandiimidaten mit wäßriger Cyanamidlösung oder Chlorcyan in einem Zwei- oder Dreistufenprozeß hergestellt werden können (EP-A-0 024 200), wobei die Bildung und anschließende Isolierung eines N-Cyanoimidates als Zwischenprodukt als charakteristische Verfahrensmerkmale anzusehen sind. Die hierbei erhältlichen Ausbeuten für das Endprodukt sind jedoch unbefriedigend. Weitere Nachteile des genannten Verfahrens sind: die Bildung nicht unerheblicher Mengen an Nebenprodukten, die Notwendigkeit der Reaktionsführung in einem engen pH-Bereich und die zusätzliche Verwendung einer Base. Außerdem ist von Nachteil, daß die während der Umsetzung gebildeten anorganischen Salze, speziell beim Einsatz von wäßriger Cyanamidlösung, in gelöster Form ins Abwasser gelangen, aus welchem sie durch zusätzliche Maßnahmen entfernt werden müssen.

Überraschenderweise verläuft das erfindungsgemäße Verfahren demgegenüber in einem verfahrenstechnisch einfachen Einstufenprozeß ohne isolierbare Zwischenprodukte, der eine Reaktionsführung ohne pH-Kontrolle gestattet. Nebenprodukte werden nur in untergeordnetem Maß gebildet.

Beim erfindungsgemäßen Verfahren greift der Imidstickstoff am Cyansäureester an, ohne daß dabei ein Cyanimidat als Zwischenstufe durchlaufen oder gebildet wird. Die hohe Selektivität bei der Bildung von Verbindungen der Formel I und die damit verbundenen hohen Ausbeuten waren nicht zu erwarten.

Im Verlauf der Reaktion werden keine Salze gebildet, die zu einer Abwasserbelastung führen könnten. Neben den bereits beschriebenen verfahrenstechnischen und wirtschaftlichen Vorteilen, ist dieser Aspekt aus ökologischer Sicht als fortschrittlich anzusehen. Nach Beendigung der Reaktion, Extraktion des gebildeten Alkohols ($R^3OH$) und Abdestillieren des Lösungsmittel, welches nahezu quantitativ zurückgewonnen wird, verbleibt des Endprodukt in hoher Reinheit.

Das erfindungsgemäße Verfahren wird zweckmäßigerweise so durchgeführt, daß man das Propandiimidat als Mono- oder Disalz oder als Bisimidat isoliert, in Lösung oder als Suspension mit dem Cyansäureester der Formel III bei Reaktionstemperaturen von 0-180 °C, vorzugsweise 20-140 °C, umsetzt. Als Salze des Propandiimidates werden bevorzugt solche der Fluor-, Chlor- oder Bromwasserstoffsäure oder der Schwefel- oder Phosphorsäure eingesetzt. Haloalkylreste für $R^1$, $R^2$, $R^3$ sind beispielsweise $CH_2Cl$, $CHCl_2$, $CCl_3$, $CH_2CH_2Cl$ oder $CH_2CF_3$. Vorzugsweise bedeuten X und Y Sauerstoff. $R^1$ und $R^2$ bedeuten vorzugsweise ($C_1$-$C_4$)Alkyl, insbesondere Methyl. $R^3$ bedeutet vorzugsweise Phenyl, das bis zu 3 Substituenten, insbesondere 1 oder 2 Substituenten, aus den Resten Chlor, Methyl und Methoxy aufweisen kann, oder $\beta$-Naphthyl.

Als inerte organische Lösungsmittel sind solche geeignet, die unter den jeweiligen Reaktionsbedingungen inert sind. Beispielsweise können als inerte organische Lösungsmittel Alkohole wie Methanol, Ethanol, und Propanol, Ketone wie Aceton und Methylisobutylketon, Ether wie Diethylether, Dioxan und Tetrahydrofuran, Ester wie Methylacetat, Ethylacetat und Butylacetat, Kohlenwasserstoffe wie Toluol, Xylol, Hexan und Cyclohexan, Nitrole wie Acetonitril und halogenierte Kohlenwasserstoffe wie Chloroform und Methylenchlorid oder Mischungen der aufgeführten Lösungsmittel verwendet werden.

Um störende Einflüsse von Sauerstoff auf die Reaktion zu vermeiden, ist es zweckmäßig, unter Inertgasatmosphäre, beispielsweise unter Stickstoff, zu arbeiten.

Die Verbindungen der Formel II können nach bekannten Methoden hergestellt werden (S.M. McElvain and I.D. Schroeder, JACS 71, 40 (1949); B. Harstun, DE-OS 2426913).

Das Monosalz und das Bisimidat dieser Verbindungen können aus dem entsprechenden Disalz durch Umsetzung mit Basen wie Alkali- und Erdalkalihydroxiden, -carbonaten, -hydrogencarbonaten oder -alkoholaten in einem inerten Lösungsmittel hergestellt werden.

Nachfolgende Beispiele sollen das erfindungsgemäße Verfahren näher erläutern:

**Beispiel 1**

Zu 800 ml Diethylether werden bei Raumtemperatur unter Stickstoffüberlagerung 52 g Dimethylpropandiimidat und 49 g Phenylcyanat gegeben. Nach 5 Stunden Erwärmen auf 36 °C wird das Phenol mit 10 %iger Natronlauge ausgewaschen und das Lösungsmittel abdestilliert.

Es verbleiben 55 g Produkt, was einer Ausbeute von 89 % der Theorie entspricht.
Der Schmelzpunkt beträgt 93-94 °C.

**Beispiel 2**

Zu 800 ml Methylisobutylketon werden unter Stickstoffüberlagerung bei 50 °C 49 g Phenylcyanat und 52 g Dimethylpropandiimidat gegeben und für 5 Stunden reagieren gelassen. Das entstehende Phenol wird mit Natronlauge extrahiert und das Lösungsmittel abdestilliert. Es verbleiben 56,4 g Produkt, was einer Ausbeute von 91 % der Theorie entspricht. Der Schmelzpunkt beträgt 92-94 °C.

**Beispiel 3**

In einer Lösung von 500 ml Aceton werden unter Stickstoffüberlagerung bei 40 °C 52 g Dimethylpropandiimidat und 75,2 g 2,4-Dichlorphenylcyanat gegeben und 5 Stunden bei 40 °C reagieren gelassen. Nach Extrahieren von 2,4-Dichlorphenol wird das Lösungsmittel abdestilliert.
Es verbleiben 54,5 g Produkt, was einer Ausbeute von 88 % der Theorie entspricht.
Der Schmelzpunkt ist 92-94 °C.

**Beispiel 4**

Zu 500 ml Essigsäuremethylester werden 59 g 2,4-Dimethylphenylcyanat und 67 g Dimethylpropandiimidatmonohydrochlorid gegeben und 5 Stunden bei 50 °C reagieren gelassen. Nach Extrahieren von 2,4-Dimethylphenol mit Natronlauge wird das Lösungsmittel abdestilliert.
Es verbleiben 53 g Produkt, was einer Ausbeute von 86 % der Theorie entspricht.

Der Schmelzpunkt beträgt 92-93 °C.

**Beispiel 5**

Zu 500 ml Toluol werden bei 70 °C 52 g Dimethylpropandiimidat und 63 g β-Naphthylcyanat gegeben und 5 Stunden bei 70 °C reagieren gelassen. Nach Entfernen des β-Naphthols wird das Lösungsmittel abdestilliert. Es verbleiben 55,8 g Produkt, was einer Ausbeute von 90 % der Theorie entspricht.

**Beispiel 6**

Zu 800 ml Toluol werden unter Stickstoffüberlagerung bei 110 °C 52 g Dimethylpropandiimidat und 67,0g 2-Methyl-4-chlorphenylcyanat gegeben und 5 Stunden bei 110 °C reagieren gelassen. Nach Extraktion von 2-Methyl-4-chlorphenol wird das Lösungsmittel abdestilliert. Es verbleiben 55,2 g Produkt, was einer Ausbeute von 89,2 % der Theorie entspricht.
Der Schmelzpunkt des Produkts ist 92-94 °C.

**Beispiel 7**

Zu 800 ml Toluol werden unter Stickstoffüberlagerung bei 90 °C 52 g Dimethylpropandiimidat und 73,4g 4-Chlor-2-methoxyphenylcyanat gegeben und 6 Stunden bei 90 °C reagieren gelassen. Nach Extraktion von 4-Chlor-2-methoxyphenol wird das Lösungsmittel abdestilliert. Es verbleiben 53,2 g Produkt (Ausbeute 86 % d. Th.) mit einem Schmelzpunkt von 92-93 °C.

**Beispiel 8-19**

Analog zu den in den Beispielen 1-7 beschriebenen Verfahrensweisen lassen sich beispielsweise folgende Verbindungen der Formel I herstellen:

| Beispiel | X | Y | $R^1$ | $R^2$ |
|---|---|---|---|---|
| 8 | O | O | $C_2H_5$ | $C_2H_5$ |
| 9 | S | S | $C_2H_5$ | $C_2H_5$ |
| 10 | S | S | $CH_3$ | $CH_3$ |
| 11 | O | O | $CH(CH_3)-CH_3$ | $CH(CH_3)-CH_3$ |
| 12 | O | O | $CF_3$ | $CF_3$ |
| 13 | O | S | $CH_2Cl$ | $CH_2Cl$ |
| 14 | S | S | $CH_2OCH_3$ | $CH_2OCH_3$ |
| 15 | O | O | $CH_2Cl$ | $CH_2Cl$ |
| 16 | S | O | $CHCl_2$ | $CHCl_2$ |
| 17 | O | O | $CH_2OC_2H_5$ | $CH_2OC_2H_5$ |
| 18 | O | S | $CH_2OC_2H_5$ | $CH_2OC_2H_5$ |
| 19 | S | S | $CF_3$ | $CF_3$ |

## Patentansprüche

**1.** Verfahren zur Herstellung von Verbindungen der Formel I

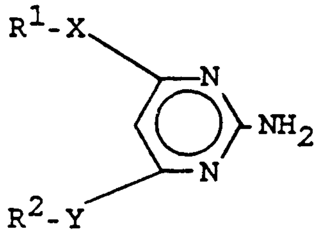

I,

worin

X und Y unabhängig voneinander Sauerstoff oder Schwefel und
$R^1$ und $R^2$ unabhängig voneinander $(C_1\text{-}C_4)$Alkyl, $(C_1\text{-}C_4)$Alkoxy-$(C_1\text{-}C_2)$-alkyl oder Halo$(C_1\text{-}C_4)$-alkyl bedeuten,

durch Umsetzung eines Propandiimidates der Formel II oder eines seiner Salze,

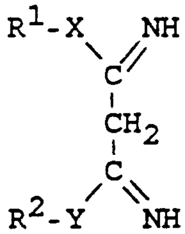

II

worin $R^1$ und $R^2$ wie in Formel I definiert sind, mit einem Cyansäurederivat,
dadurch gekennzeichnet, daß man mit einem Cyansäureester der Formel III als Cyansäurederivat,

$R^3 - O - C \equiv N$ III

worin
$R^3$ Phenyl oder Phenyl, das durch 1, 2 oder 3 Reste aus den Resten Halogen, $(C_1\text{-}C_4)$Alkoxy und $(C_1\text{-}C_4)$Alkyl substituiert ist, oder $\alpha$- oder $\beta$-Naphthyl bedeutet,

in einem inerten organischen Lösungsmittel bei Reaktionstemperaturen von 0-180 °C umsetzt.

2. Verfahren gemäß Anspruch 1, dadurch gekennzeichnet, daß in Formel I X und Y jeweils Sauerstoff und $R^1$ und $R^2$ jeweils $(C_1\text{-}C_4)$Alkyl bedeuten.

3. Verfahren gemäß Anspruch 1 oder 2, dadurch gekennzeichnet, daß man als inertes organisches Lösungsmittel einen Alkohol, ein Keton, einen Ether, einen Ester, einen Kohlenwasserstoff, einen halogenierten Kohlenwasserstoff, ein Nitril oder eine Mischung aus wenigstens zwei der vorstehenden Lösungsmittel verwendet.

4. Verfahren gemäß einem oder mehreren der Ansprüche 1-3, dadurch gekennzeichnet, daß als Lösungsmittel Diethylether, Aceton, Methylisobutylketon, Essigsäuremethylester, Toluol oder eine Mischung aus wenigstens zwei dieser Lösungsmittel verwendet wird.

5. Verfahren gemäß einem oder mehreren der Ansprüche 1-4, dadurch gekennzeichnet, daß man als Salze der Verbindung der Formel II solche der Fluor-, Chlor-, oder Bromwasserstoffsäure oder der Schwefel- oder Phosphorsäure verwendet.

6. Verfahren gemäß einem oder mehreren der Ansprüche 1 bis 4, dadurch gekennzeichnet, daß $R^3$ Phenyl oder Phenyl, das 1 oder 2 Substituenten aus den Resten Chlor, Methyl und Methoxy aufweist, oder $\beta$-Naphthyl bedeutet.

7. Verfahren gemäß einem oder mehreren der Ansprüche 1-6, dadurch gekennzeichnet, daß man bei Reaktionstemperaturen von 20-140 °C arbeitet.

8. Verfahren gemäß einem oder mehreren der Ansprüche 1-7, dadurch gekennzeichnet, daß man die Reaktion unter Inertgasatmosphäre durchführt.

## Claims

1. A process for the preparation of compounds of the formula I

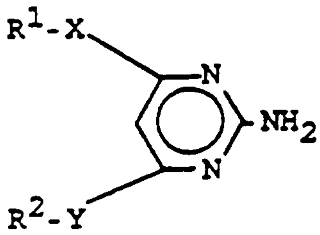

I,

in which
X and Y, independently of one another, are oxygen or sulfur,
$R^1$ and $R^2$, independently of one another, are $(C_1\text{-}C_4)$-alkyl, $(C_1\text{-}C_4)$alkoxy-$(C_1\text{-}C_2)$-alkyl or halo$(C_1\text{-}C_4)$-alkyl,
by reacting a propanediimidate of the formula II

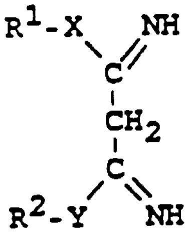

II

in which $R^1$ and $R^2$ are as defined in formula I, or a salt thereof, with a cyanic acid derivative, characterized by carrying out the reaction using, as the cyanic acid derivative, a cyanic ester of the formula III

$R^3 - O - C \equiv N$   III

in which
$R^3$ is phenyl or phenyl which is substituted by 1,2 or 3 radicals from the radicals halogen, $(C_1\text{-}C_4)$-alkoxyand $(C_1\text{-}C_4)$alkyl, or is $\alpha$- or $\beta$-naphthyl,
in an inert organic solvent at reaction temperatures of 0-180 °C.

2. The process as claimed in claim 1, wherein, in the formula I, X and Y are each oxygen and $R^1$ and $R^2$ are each $(C_1\text{-}C_4)$alkyl.

3. The process as claimed in claim 1 or 2, wherein the inert organic solvent used is an alcohol, a ketone, an ether, an ester, a hydrocarbon, a halogenated hydrocarbon, a nitrile or a mixture of at least two of the above solvents.

4. The process as claimed in one or more of claims 1-3, wherein the solvent used is diethyl ether, acetone, methyl isobutyl ketone, methyl acetate, toluene or a mixture of at least two of these solvents.

5. The process as claimed in one or more of claims 1-4, wherein the salts of the compound of the formula II are those of hydrofluoric, hydrochloric or hydrobromic acid or of sulfuric or phosphoric acid.

6. The process as claimed in one or more of claims 1 to 4, wherein $R^3$ is phenyl or phenyl containing 1 or 2 substituents from the radicals chlorine, methyl and methoxy or is $\beta$-naphthyl.

7. The process as claimed in one or more of claims 1-6, wherein the reaction is carried out at temperatures of 20-140 °C.

8. The process as claimed in one or more of claims 1-7, wherein the reaction is carried out under an inert-gas atmosphere.

**Revendications**

1. Procédé pour préparer des composés de formule I

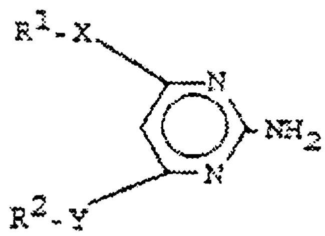

I

[dans laquelle

X et Y représentent chacun, indépendamment l'un de l'autre, un atome d'hydrogène ou de soufre, et

$R^1$ et $R^2$ représentent chacun, indépendamment l'un de l'autre, un groupe alkyle (en $C_1$ à $C_4$), alcoxy (en $C_1$ à $C_4$)-alkyle (en $C_1$ ou $C_2$) ou halogéno-alkyle (en $C_1$ à $C_4$)], par réaction d'un propane diimidate de formule II ou d'un de des sels :

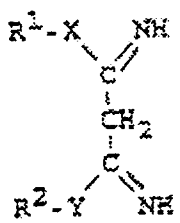

II

[dans laquelle $R^1$ et $R^2$ sont tels que définis pour la formule I] avec un dérivé de l'acide cyanique, procédé caractérisé en ce qu'on fait réagir un ester de l'acide cyanique, de formule (III) comme dérivé de l'acide cyanique :

$R^3 - O - C \equiv N$ III,

[dans laquelle

$R^3$ représente un groupe phényle, ou bien un groupe phényle qui est substitué par 1,2, ou 3 restes choisis parmi les restes halogéno, alcoxy (en $C_1$ à $C_4$) et alkyle (en $C_1$ à $C_4$) ou un groupe $\alpha$ ou $\beta$-naphtyle], dans un solvant organique inerte à des températures de réaction de 0 à 180 °C.

2. Procédé selon la revendication 1, caractérisé en ce que, dans la formule (I), X et Y représentent chacun un atome d'oxygène et $R^1$ et $R^2$ représentent chacun un groupe alkyle (en $C_1$ à $C_4$).

3. Procédé selon la revendication 1 ou 2, caractérisé en ce qu'on utilise comme solvant organique inerte un alcool, une cétone, un éther, un ester, un hydrocarbure, un hydrocarbure halogéné, un nitrile ou un mélange d'au moins deux des solvants précités.

4. Procédé selon une ou plusieurs des revendications 1 à 3, caractérisé en ce qu'on utilise comme solvant l'éther diéthylique, l'acétone, la méthylisobutylcétone, l'ester méthylique de l'acide acétique, le

toluène ou un mélange d'au moins 2 de ces solvants.

**5.** Procédé selon une ou plusieurs des revendications 1 à 4, caractérisé en ce qu'on utilise comme sels du composé de formule (II) les sels de l'acide fluorhydrique, de l'acide chlorhydrique ou de l'acide bromhydrique ou bien de l'acide sulfurique ou de l'acide phosphorique.

**6.** Procédé selon une ou plusieurs des revendications 1 à 4, caractérisé en ce que $R^3$ représente un groupe phényle ou bien un groupe phényle (qui présente un ou deux substituants choisis parmi les reste chloro, méthyle et méthoxy) ou un groupe $\beta$-naphtyle.

**7.** Procédé selon une ou plusieurs des revendications 1 à 6, caractérisé en ce qu'on travaille à des températures de réaction de 20 à 140 °C.

**8.** Procédé selon une ou plusieurs des revendications 1 à 7, caractérisé en ce qu'on effectue la réaction en atmosphère de gaz inerte.